(19) Europäisches Patentamt / European Patent Office / Office européen des brevets

(11) **EP 4 382 614 A1**

(12) **EUROPEAN PATENT APPLICATION**

(43) Date of publication:
**12.06.2024 Bulletin 2024/24**

(21) Application number: **22211669.1**

(22) Date of filing: **06.12.2022**

(51) International Patent Classification (IPC):
*C12Q 1/6806* (2018.01)      *C12Q 1/6869* (2018.01)
*G01N 21/64* (2006.01)

(52) Cooperative Patent Classification (CPC):
(C-Sets available)
**C12Q 1/6806; C12Q 1/6869;** G01N 21/6408;
G01N 21/6452; G01N 2021/6432      (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Gnothis Holding AG
6330 Cham (CH)**

(72) Inventors:
• **EDMAN, Lars
6330 Cham (CH)**
• **LUNDBERG, Oscar
6330 Cham (CH)**
• **REDIN, David
6330 Cham (CH)**

(74) Representative: **Weickmann & Weickmann
PartmbB
Postfach 860 820
81635 München (DE)**

(54) **HIGH YIELD SUPPORT**

(57)    The present disclosure relates to supports comprising a substrate and a plurality of sample spots on the surface of the support, wherein the sample spots are spatially separated from another, and wherein a single biomolecule is immobilized on a single sample spot, and to methods of manufacturing such supports.

EP 4 382 614 A1

(52) Cooperative Patent Classification (CPC): (Cont.)

C-Sets
**C12Q 1/6806, C12Q 2535/122, C12Q 2565/50;**
**C12Q 1/6869, C12Q 2535/122, C12Q 2565/50**

**Description**

The present disclosure relates to supports comprising a substrate and a plurality of sample spots on the surface of the support, wherein the sample spots are spatially separated from another, and wherein a single biomolecule is immobilized on a single sample spot, and to methods of manufacturing such supports.

Background

Sequencing of the human genome or the genome of other organisms and the determination and comparison of individual sequence variants requires the provision of sequencing methods which firstly are fast and secondly can be employed routinely and cost-effectively.
The high demand for cost-efficient sequencing has driven the development of high-throughput sequencing technologies that parallelize the sequencing process producing a plurality of sequences concurrently. Examples of these sequencing technologies are massively parallel signature sequencing (Lynx Therapeutics), polony sequencing (Life Technologies), 454 pyrosequencing (Roche Diagnostics), illumina sequencing (Solexa Inc.), sequencing by ligation (Life Technologies), ion torrent semiconductor sequencing (Life Technologies) or DNA nanoball sequencing (Complete Genomics). These technologies allow rapid analysis of a consensus sequence in a nucleic acid population. Mutations existing in minority sequences in the nucleic acid population to be analysed, e.g., in a minority of cellular genomes, however, will not be detected since they are obscured by the majority of other sequences present in the population.
In order to address this issue, single molecule sequencing processes in several different formats have been developed. Typically, nucleic acid-polymerizing enzymes and/or nucleic acid-degrading enzymes and fluorescence-labelled nucleic acids and/or nucleotide building blocks are used for individually determining the sequence of a single nucleic acid molecule on the basis of a time-dependent change in fluorescence when nucleotide building blocks are incorporated into or cleaved off from a nucleic acid molecule. Single molecule sequencing processes and devices adapted for performing such processes are e.g., described in co-owned applications WO 2002/097406, WO 2003/052137, WO 2006/013110, WO 2013/131888, WO 2015/104245, WO 2017/001407, and WO 2018/104301.
In several of known processes and devices, nucleic acid-degrading enzyme molecules and/or nucleic acid-synthesizing enzyme molecules or complexes of such enzyme molecules with nucleic acid molecules are provided in an immobilized form on a solid support.
While it is assumed that there is a single molecule situated on each sample spot the actual efficiency of the single molecule immobilization process is not described.
It was an object of the present disclosure to provide supports suitable for an analysis of single molecule events, e.g., for a sequence analysis of a single nucleic acid molecule, comprising an increased yield of sample spots where one and only one biomolecule is immobilized.

Summary of the Invention

In a first aspect, the present disclosure relates to a method for preparing a support for analyzing a single molecule event comprising the steps:

(a) providing a support comprising a substrate and a plurality of sample spots on the surface of the support, wherein said sample spots are spatially separated from another and wherein said sample spots are adapted for the immobilization of one single biomolecule on one sample spot, and
(b) incubating said sample spots on said support with biomolecules under conditions that allow a saturating immobilization of said biomolecules to said sample spots.

A further aspect of the present disclosure relates to a support comprising a substrate and a plurality of sample spots on the surface of the support, wherein the sample spots are spatially separated from another, and wherein a biomolecule is immobilized on a sample spot, wherein a single biomolecule is immobilized to a single sample spot in at least about 50% of said sample spots.
A further aspect of the present disclosure relates to the use of the above support for analyzing an event at the single sample spot wherein said event is associated with emission of an electromagnetic radiation. In a particular embodiment, the event is a single molecule event.
A further aspect of the present disclosure relates to a method for analyzing a single molecule event comprising:

(i) providing the above support, wherein a single biomolecule is immobilized on a single sample spot in at least about 50% of the sample spots, and
(ii) analyzing an event, associated with the single biomolecule, by detecting electromagnetic radiation from an individual sample spot.

In a particular embodiment, the single molecule event comprises a sequence analysis of a single nucleic acid molecule.
A further aspect of the present disclosure relates to a device for analyzing a single molecule event comprising:

(i) the above support, wherein a single biomolecule is immobilized on a single sample spot in at least about 50% of the sample spots,

(ii) means for illuminating at least one sample spot on the support with radiation, and
(iii) means for analyzing an event associated with the single biomolecule, by detecting electromagnetic radiation from an individual sample spot.

In a particular embodiment, the device is adapted for the sequence analysis of a single nucleic acid molecule.

Items of the Specification

1. A method for preparing a support for analyzing a single molecule event comprising the steps:

(c) providing a support comprising a substrate and a plurality of sample spots on the surface of the support, wherein said sample spots are spatially separated from another and wherein said sample spots are adapted for the immobilization of one single biomolecule on one sample spot, and
(d) incubating said sample spots on said support with biomolecules under conditions that allow a saturating immobilization of said biomolecules to said sample spots.

2. The method of item 1, wherein a single biomolecule is immobilized on a single sample spot in at least about 50% of said sample spots on said support.

3. The method of item 1 or 2, wherein a single biomolecule is immobilized on a single sample spot in at least about 60%, at least about 70% at least about 80%, at least about 90% or at least about 99% and up to 99.99% of said sample spots on said support.

4. The method of any one of the preceding items, wherein the support comprises at least 10, at least 100, at least 1,000, at least $10^4$ , at least $10^5$, at least $10^6$, at least $10^7$ or at least $10^8$ sample spots.

5. The method of any one of the preceding items, wherein sample spots are provided on the surface of the support so that the distance between adjacent sample spots is about 1 nm to about 5000 nm, e.g., about 2 nm to about 2000 nm.

6. The method of any one of the preceding items, wherein sample spots are provided that have a predetermined size based on the specific biomolecule to be immobilized thereon.

7. The method of item 6, wherein the sample spots have a size of about 5 nm to about 10 nm, particularly a size of about 6 nm to about 8 nm and more particularly about 7 nm for the immobilization of one single biomolecule on one said sample spot wherein said single biomolecule is polypeptide and/or a nucleic acid molecule.

8. The method of item 7, wherein the biomolecule is a nucleic acid-polymerizing enzyme, particularly a DNA or RNA polymerase, and/or a nucleic acid molecule, particularly a DNA or RNA molecule.

9. The of any one of the preceding items, wherein sample spots are provided that have a standard deviation around their average size of 20% or less.

10. The method of any one of the preceding items, wherein said sample spots are adapted in their surface chemistry for the immobilization of one single biomolecule on one said sample spot.

11. The method of item 10, wherein the surface of the sample spots comprises at least one functional anchor molecule, which comprises a moiety suitable for the direct or indirect attachment of a biomolecule.

12. The method of item 11, wherein said functional anchor molecule is attached to the surface of said sample spots with sulfur-containing groups, e.g., thiol groups.

13. The method of item 11 or 12, wherein the sample spots comprise a metal surface, particularly an Au or Au/Pd surface.

14. The method of any one of the preceding items, wherein the conditions that allow a saturating immobilization of said biomolecules to said sample spots comprise adapting at least one of the following parameters:

(i) the concentration of biomolecules;
(ii) the incubation time;
(iii) the incubation temperature;
(iv) any combination of (i), (ii), and/or (iii).

15. The method of any one of the preceding items, wherein the conditions that allow a saturating immobilization of said biomolecules to said sample spots comprise carrying out the incubation step under conditions wherein the amount of biomolecules is higher than the amount of available sample spots.

16. The method of item 14, wherein the amount of biomolecules is at least 10-times higher than the amount of available sample spots.

17. The method of any one of the preceding items, wherein the support is at least substantially planar.

18. The method of any one of the preceding items, wherein the support is structured.

19. The method of any one of the preceding items, wherein the substrate is optically transparent.

20. The method of any one of the preceding items, wherein the substrate comprises a material having a refractive index of at least 1.01.

21. The method of any one of the preceding items, wherein the substrate comprises a non-electrically conductive material, e.g., selected from the group consisting of silica, quartz, and glass.

22. The method of any one of the preceding items, wherein the substrate has a thickness of about 10 $\mu$m to about 5 mm, particularly about 20 $\mu$m to about 2 mm.

23. The method of any one of the preceding items, wherein a sample spot comprises at least one electrically conductive material, e.g., a metal including a pure metal or a combination of metals, e.g., an alloy or mixture of a plurality of different metals.

24. The method of any one of the preceding items, wherein the sample spot comprises a metal is capable of forming a bond with a sulfur-containing group, e.g., in the form of a thiol or a disulfide.

25. The method of any one of the preceding items, wherein the at least metal has a positive electrochemical potential.

26. The method of any one of the preceding items, wherein the at least one metal is selected from Au, Cu, Ni, Pt, Pd, Rh, Ir, Os, Ru and any combination comprising at least two of said metals.

27. The method of any one of the preceding items, wherein the at least one sample spot comprises at least one metal oxide such as $TiO_2$, NiO, or ITO.

28. The method of any one of the preceding items, wherein the biomolecule is selected from the group consisting of polypeptides, nucleic acids, carbohydrates, and any combination thereof.

29. The method of any one of the preceding items, wherein the biomolecule is a nucleic acid-polymerizing enzyme, particularly a DNA polymerase or an RNA-polymerase, or a nucleic acid-polymerizing molecule complex, particularly a DNA- or RNA-polymerizing complex comprising a nucleic acid-polymerizing enzyme and a nucleic acid molecule.

30. The method of any one of the preceding items, wherein the biomolecule is a DNA polymerase having a DNA binding cleft, particularly a Family A DNA polymerase including, but not limited to a Klenow, Taq or T7 DNA polymerase or any genetically modified version thereof, or a Family B polymerase including, but not limited to a therminator, Phi29, RB-69 or T4 DNA polymerase or any genetically modified version thereof.

31. The method of any one of the preceding items, wherein the biomolecule is a gene editing enzyme, particularly a Cas nuclease such as a Cas9 nuclease or any genetically modified version thereof, or a gene editing complex comprising a gene editing enzyme and nucleic acid molecule, e.g., a guide RNA and/or a target nucleic acid.

32. A support comprising a substrate and a plurality of sample spots on the surface of the support, wherein the sample spots are spatially separated from another, and wherein a biomolecule is immobilized on a sample spot, wherein a single biomolecule is immobilized on a single sample spot in at least about 50% of said sample spots.

33. The support of item 32 obtainable by the method of any one of items 1-31.

34. Use of the support of item 32 or 33 for analyzing a single molecule event.

35. The use of item 34, wherein the single molecule event comprises a sequence analysis of a single nucleic acid molecule.

36. A method for analyzing a single molecule event comprising:

(i) providing a support of any one of items 32-33, wherein a single biomolecule is immobilized on a single sample spot in at least about 50% of said sample spots, and
(ii) analyzing an event, associated with said single biomolecule, by detecting electromagnetic radiation from an individual sample spot.

37. The method of item 36, comprising separately detecting electromagnetic radiation from a plurality of individual sample spots in parallel.

38. The method of item 36 or 37, wherein the single molecule event comprises a sequence analysis of a single nucleic acid molecule.

39. A device for analyzing a single molecule event comprising:

(i) a support of any one of items 32-33, wherein a single biomolecule is immobilized on a single sample spot in at least about 50% of said sample spots,

(ii) means for illuminating at least one sample spot on the support with radiation, and

(iii) means for analyzing an event associated with said single biomolecule, by detecting electromagnetic radiation from an individual sample spot.

40. The device of item 39 adapted for separately detecting electromagnetic radiation from a plurality of individual sample spots in parallel.

41. The device of item 39 or 40 adapted for the sequence analysis of a single nucleic acid molecule.

Detailed Description

The present disclosure relates to a method for preparing a support for analyzing a single molecule event and a support obtainable by the method. The support comprises a substrate and a plurality of sample spots on the support surface that are spatially separated from another. In certain embodiments, the support surface is formed by the substrate and the sample spots. In certain embodiments, the substrate forms a contiguous area in which the sample spots are distributed. An individual sample spot on the support surface is surrounded by the substrate, which differs from the sample spot, e.g., in respect of the material and/or the surface. Typically, the substrate is adapted for inhibiting and/or blocking adhesion of biomolecules such as polypeptides whereas the sample spots are adapted for allowing adhesion of desired biomolecules.

The support provided by the present disclosure comprises an increased yield of sample spots where one and only one biomolecule is immobilized. The present inventors have identified means how one and only one single molecule will be located on each sample spot, and how a theoretical yield of 100% (and in practice close to 100%, e.g., up to 99.9% or 99.99%) can be achieved.

In certain embodiments, a single biomolecule is immobilized on a single sample spot in a yield of at least about 50% of said sample spots on said support. In particular embodiments, a single biomolecule is immobilized on a single sample spot in at least about 60%, at least about 70% at least about 80%, at least about 90% or at least about 99% and up to 99.99% of said sample spots on said support. The yield of single biomolecules immobilized on a single sample spot for a particular support can be done by several spectroscopic and/or microscopic methods In certain embodiments, determination of sample spots occupied by single biomolecules is carried out by fluorescence spectroscopy if the bound molecules are fluorescent or if they are used for catalyzing a reaction in which a fluorescent product is formed. In further embodiments, determination of sample spots occupied by single biomolecules is carried out by atomic force microscopy.

The percentage P of sample spots occupied by single biomolecules is calculated as follows:

$$P = n/N$$

wherein n is the number of individual sample spots occupied by a single biomolecule, and N is the total number of sample spots on the support.

The term "single biomolecule" encompasses a single molecular entity such as a polypeptide or a complex consisting of a plurality of individual units, e.g., individual molecular entities wherein the individual units form together a functional biological moiety.

In certain embodiments, the support is a substantially planar support, i.e., it does not comprise elevations or recesses of about 1000 nm or more or of about 100 nm or more. In further embodiments, the support is a structured support, e.g., a support comprising recesses such as wells that may have a volume of about $5 \times 10^{-24}$ liters to about $1 \times 10^{-15}$ liter, or pillars of height of about 1 nm to 500 nm. In principle, the support may have any design, as long as a reaction space can be formed which enables the occurrence of a single molecule event on the sample spot where the single biomolecule is immobilized.

In certain embodiments, the substrate is an optically transparent material, i.e., a material that is substantially transparent for electromagnetic radiation, e.g., radiation in the visible range and/or radiation in the near-infrared range. In certain embodiments, the substrate comprises a material having an absolute refractive index of at least 1.01, e.g., about 1.5 to about 3 in the visible range or about 1.5 to about 4 in the near-infrared range. In further embodiments, the substrate is an optically opaque material, e.g., a metal or semi-metal such as silicon.

In particular embodiments, the substrate comprises a non-electrically conductive material. Specific examples are glass, quartz, plastic, metal oxide-based materials, e.g., silicon dioxide-based materials such as glass, silica, or quartz, or composites comprising said materials. In further embodiments, the substrate comprises an electrically conductive material, e.g., an optically transparent material such as indium tin oxide.

Typically, the substrate has a thickness of about 10 $\mu$m to about 5 mm, particularly about 20 $\mu$m to about 2 mm.

In certain embodiments, the substrate is coated with a layer of diamond-like carbon and/or amorphous carbon (also designated as "carbon film") as described in copending application US 63/382,624, the content of which is herein incorporated by reference. The carbon film may be deposited on the substrate by physical vapor deposition, chemical vapor deposition (CVD) or atomic layer deposition (ALD) techniques. The carbon film may form a continuous layer on the surface of the support except for the area of the sample spots. In certain embodiments, the carbon film is fluorinated. Fluorine atoms may be introduced by known procedures, e.g., during DLC deposition by CVD or by exposure to a fluorine-containing plasma from fluorocarbon-based gases or the like (e.g., $C_4F_8$, $CHF_3$, $NF_3$, or $SF_6$).

In certain embodiments, the carbon film including a fluorinated carbon film has a thickness of about 0.3 nm to about 200 $\mu$m, particularly about 10 nm to about 100 $\mu$m, and more particularly about 1 $\mu$m to about 50 $\mu$m.

In certain embodiments, the surface of the substrate is coated with an organic passivating reagent, e.g., a polyethylene glycol containing reagent that inhibits and/or blocks adhesion of biomolecules such as proteins and/or nucleic acids.

The surface of the support comprises a plurality of sample spots that are spatially separated from another by the substrate surface. The sample spots are adapted for attachment of single biomolecules. In certain embodiments, the support comprises a plurality of sample spots, e.g., at least 10, at least 100, at least 1,000, at least $10^4$, at least $10^5$, at least $10^6$, at least $10^7$ or at least $10^8$ sample spots. In certain embodiments, the support may comprise up to $10^9$ or even more sample spots on a single support. In certain embodiments, the sample spots are provided on the surface of the support so that the distance between adjacent sample spots is about 1 nm to about 5000 nm, e.g., about 2 nm to about 2000 nm. The distribution of the sample spots on the support may be even or uneven, e.g., clustered in groups.

In certain embodiments, a sample spot comprises or consists of at least one electrically conductive material, e.g., a metal including a single metal or a combination of metals, e.g., an alloy or mixture of a plurality of different metals. For example, a metal that is capable of attachment to a sulfur containing moiety, e.g., in the form of a thiol or a disulfide, or a metal that is capable of attachment to a chelating moiety, e.g., a poly-histidine tag, is suitable. In certain embodiments, the metal has a positive electrochemical potential. Specific examples of suitable metals include but are not limited to Au, Cu, Ni, Pt, Pd, Rh, Ir, Os, Ru, and any combination thereof comprising at least two of said metals.

In certain embodiments, a sample spot comprises or consists of at least one at least one metal oxide including a single metal oxide or a combination of metal oxides. For example, a metal oxide that is capable of attachment to a phosphorus containing moiety, e.g., in the form of a phosphonic acid or phosphonic acid ester, or a metal oxide that is capable of attachment to a chelating moiety, e.g., a poly-histidine tag, is suitable. Specific examples of suitable metal oxides include $TiO_2$ and NiO.

In further embodiments, a sample spot comprises or consists of at least one non-electrically conductive material.

Sample spots may be prepared by vapour deposition of metals, which are vaporized on the support covered by a grid mask, which may be produced by electron beam lithography or equivalent technologies. The size of holes in the grid mask may correspond to the size of the spots on the support surface. Alternatively, the spots on the support may be prepared by site-specific deposition of nanoparticles, e.g., having a size of 2-10 nm, by precision pipetting of particles on the support, particularly on a support having a planar surface.

The sample spot may have a size that is suitable for the attachment of single biomolecules as defined herein. In particular embodiments, a sample spot has a diameter of about 1 nm to about 30 nm, particularly a diameter of about 2 nm to about 20 nm.

In certain embodiments, the sample spot is a separate object on the surface of the substrate. In certain embodiments, the sample spot has a lower face proximal to the substrate and an upper face distal to the substrate, wherein the distance between the lower and upper face defines the height of the sample spot. In particular embodiments, the height is about 50 pm to about 500 nm, particularly about 100 pm to about 20 nm, and more particularly about 500 pm to about 10 nm, e.g., about 2 nm.

In order to achieve the desired high yield, the sample spots are adapted for the immobilization of one single biomolecule on one sample spot. This may be achieved by designing sample spots to which not more than one biomolecule per spot can bind due to sterical reasons, e.g., there is not space enough for more than exactly one molecule per spot.

In certain embodiments, sample spots are provided that have a predetermined size based on the specific biomolecule to be immobilized thereon. The size of the sample spot, e.g., its diameter is adapted to the size of the specific biomolecule.

In particular embodiments, the sample spots have a size of about 5 nm to about 10 nm, particularly a size of about 6 nm to about 8 nm and more particularly about 7 nm for the immobilization of one single biomolecule, e.g., a polypeptide and/or a nucleic acid molecule on one sample spot. In those embodiments, the biomolecule is a nucleic acid-polymerizing enzyme, particularly a DNA or RNA polymerase and/or a nucleic acid molecule, particularly a DNA or RNA molecule.

In certain embodiments, the sample spots have a low standard deviation around their average size thereby providing a uniform size distribution profile. This can be achieved by modern nanofabrication techniques. In particular embodiments, the standard deviation of the sample spot size around their average size is about 20% or less.

In certain embodiments, the sample spots are adapted in their surface chemistry for the immobilization of one single biomolecule on one said sample spot. In particular embodiments, the sample spots have a coating providing a limited number of binding sites for the immobilization of a biomolecule, e.g., a single binding site for a biomolecule in at least 50% of said sample spots.

In certain embodiments, the surface of the sample spots comprises at least one functional anchor molecule, which comprises a moiety suitable for the direct or indirect attachment of a biomolecule. The moiety suitable for attaching a biomolecule may e.g., be selected from biotin or Click-functional groups. In particular embodiments, the functional anchor molecules are attached to the surface of said sample spots with sulfur-containing groups, e.g., thiol groups. The surface of the metal spots may comprise a metal, e.g., as herein described supra, particularly an Au or Au/Pd surface.

In particular embodiments, the sample spots are adapted in size and surface chemistry for the immobilization of a specific single biomolecule. For example, a nucleic acid-polymerizing enzyme, particularly a DNA or RNA polymerase, may be immobilized on a sample spot having a size of about 6 nm to about 8 nm and more particularly about 7 nm and a surface, e.g., a surface of metal such as Au and/or Pd, to that anchor molecules with sulfur-containing groups, e.g., thiol groups, are attached.

According to the present disclosure, the support comprising sample spots adapted for the immobilization of a single biomolecule is incubated support with biomolecules, typically with a biomolecules in a suitable liquid medium under conditions that allow a saturating immobilization of said biomolecules on the sample spots.

A saturating immobilization means that the incubation is carried out under saturating conditions regarding the biomolecule, i.e., that binding of a biomolecule to each available sample spot on the support is favored.

In certain embodiments, the conditions that allow a saturating immobilization of biomolecules on the sample spots comprise adapting, particularly increasing at least one of the following parameters:

(v) the concentration of biomolecules;
(vi) the incubation time;
(vii) the incubation temperature;
(viii) any combination of (i), (ii), and/or (iii).

In certain embodiments, the incubation is carried out with a high concentration of biomolecules in the liquid medium. This provides a means to ensure binding of one and only one biomolecule to each spot in case the spot has been adapted for single molecule immobilization..

This is a major advantage of the present method as compared to prior art methods in which the concentration of biomolecules was kept in a very strict interval in order to optimize the number of sample spots where a single biomolecule is immobilized. In the prior art methods, the binding follows a Poissonian stochastic density which means that it will never be possible to reach a situation with one biomolecule per sample spot is bound in a majority of the sample spots. In the case of the current innovation, Poissonian statistics are avoided, and up to 100% of the spots may have one and only one biomolecule if the binding is performed under saturating conditions, e.g., by using a very high concentration of biomolecules in the medium.

In certain embodiments, the conditions that allow a saturating immobilization of said biomolecules to the sample spots comprise carrying out the incubation step under conditions wherein the amount of biomolecules is higher than the amount of available sample spots, particularly wherein the amount of biomolecules is at least 2-times, at least 5-times or at least 10-times higher than the amount of available sample spots.

The sample spots on the support are adapted for the attachment of a biomolecule such that a single biomolecule is immobilized on a single sample spot, e.g., by a covalent or non-covalent attachment. The biomolecule may be selected from polypeptides, nucleic acids, carbohydrates, and any combination thereof, e.g., a glycosylated polypeptide, or a ribonucleoprotein. In certain embodiments, the biomolecule is a complex consisting of several individual units, e.g., several polypeptide units, or several polypeptide and nucleic acid units.

In specific embodiments, the biomolecule is a nucleic acid-polymerizing enzyme, particularly a DNA polymerase or an RNA polymerase, or a nucleic acid-polymerizing molecule complex, particularly a DNA- or RNA-polymerizing complex comprising a nucleic acid-polymerizing enzyme and a nucleic acid molecule. In particular embodiments, the biomolecule is a DNA polymerase having a DNA binding cleft, particularly a Family A DNA polymerase including, but not limited to a Klenow, Taq or T7 DNA polymerase or any genetically modified version thereof, or a Family B polymerase including, but not limited to a therminator, Phi29, RB-69 or T4 DNA polymerase or any genetically modified version thereof. Reference is made in this context to US 7,745,116 B2, the content of which is herein incorporated by reference.

In further embodiments, the biomolecule is a nucleic acid-degrading enzyme, particularly an exonuclease, or a nucleic acid-degrading molecule complex, particularly a DNA- or RNA-degrading molecule complex comprising a nucleic acid-degrading enzyme and a nucleic acid molecule.

In still further embodiments, the biomolecule is a gene editing enzyme, particularly a Cas nuclease such as a Cas3, Cas9, Cas10, or Cas12 nuclease or any genetically modified version thereof, e.g., a Cas nickase, or a gene editing complex comprising a gene editing enzyme and nucleic acid molecule, e.g., a guide RNA and/or a target nucleic acid.

In certain embodiments, the biomolecule comprises at least one anchor, e.g. a single anchor attached to a single sample spots.

In certain embodiments, the biomolecule is directly attached to the sample spot, e.g., by providing a biomolecule having an anchor suitable for a direct covalent or non-covalent attachment to the surface of the sample spot, e.g., to an inorganic surface of the sample spot. In those embodiments, the anchor may comprise the reaction product of a sulfur-containing group such as a thiol group -SH, a substituted thiol group -SR, wherein R is an organic residue, e.g., a $C_1$-$C_4$ alkyl group or a disulfide group -S-S-, or a phosphorus-containing group with a metal or metal oxide surface on the sample spots. Alternatively, the anchor may comprise the reaction product of a silane group with a metal oxide, e.g., silica surface, or the reaction product of a poly(histidine) tag with a Ni or NiO surface.

In certain embodiments, the biomolecule is indirectly attached to the sample spot, e.g., by a non-covalent linkage to a coating, e.g., an organic coating on the surface of the sample spot. In those embodiments a biomolecule may be provided that has an anchor comprising an attachment amino acid such as cysteine, a modified phenylalanine, histidine, or glutamine, or an attachment tag, e.g., a biotin, hapten, poly(histidine) tag or carbohydrate group, capable of forming a linkage with a complementary moiety of a coating attached to the surface of the sample spot, e.g., streptavidin, antibody, lectin, etc.

In certain embodiments, a biomolecule may be provided that has bio-orthogonal groups, i.e., groups not occurring in biomolecules such as an azide group or an alkyne group, e.g., a terminal or strained alkyne group such as cyclooctyne. Such bio-orthogonal groups are capable of forming covalent linkages with complementary bio-orthogonal groups attached to the surface of the sample spot. In those embodiments, the anchor may comprise the reaction product of a coupling reaction between 2 bio-orthogonal reactive groups. In certain embodiments, the coupling reaction is a Click reaction, e.g., a reaction between two Click-functional groups, e.g., an azide group and an alkyne group. In certain embodiments, the anchor comprises a triazole group.

The support of the present disclosure is suitable for analyzing an event occurring on a sample spot, wherein the event is associated with the emission of an electromagnetic radiation from the sample spot. In particular embodiments, the event encompasses a reaction of a biomolecule which is associated with emission of a characteristic electromagnetic radiation. In particular embodiments, the event is a single molecule event.

In particular embodiments, the support of the present disclosure is suitable for analyzing an event, e.g., a single molecule event occurring on the at least one sample spot, particularly for separately analyzing a plurality of single molecule events each occurring on at least one sample spot and even more particularly for separately analyzing a plurality of single molecule events is analyzed in parallel. In particular embodiments, the single molecule event comprises a nucleic acid sequence determination.

A further aspect relates to a method for analyzing a single molecule event comprising:

(i) providing the above support, wherein a single biomolecule is immobilized on a single sample spot in at least about 50% of said sample spots, and
(ii) analyzing an event, associated with said single biomolecule, by detecting electromagnetic radiation from an individual sample spot.

In particular embodiments, the single molecule event comprises the sequence analysis of a single nucleic acid molecule.

A further aspect relates to a device for analyzing a single molecule event comprising:

(i) the above support, wherein a single biomolecule is immobilized on a single sample spot in at least about 50% of said sample spots,
(ii) means for illuminating at least one sample spot on the support with radiation, and
(iii) means for analyzing an event associated with said single biomolecule, by detecting electromagnetic radiation from an individual sample spot.

In particular embodiments, the device is adapted for the sequence analysis of a single nucleic acid molecule.

Methods and devices for analyzing single molecule events are e.g., disclosed in WO 2002/097406, WO 2003/052137, WO 2006/013110, WO 2013/131888, WO 2015/104245, WO 2017/001407, and WO 2018/104301, the contents of which are herein incorporated by reference.

For the analysis of a single molecule event, the biomolecules are located at the sample spots on the support. There they are in contact with a sample liquid, which contains the free reaction partners. Thereby, one or more reaction spaces are defined. Particularly at least 100, at least 1000, or at least 10 000, and up to more than $10^6$ molecules may be analysed on a single support, e.g., a single planar support.

A nucleic acid molecule whose sequence is to be determined may be selected, for example, from DNA molecules such as genomic DNA fragments, cDNA molecules, plasmids, etc., or else from RNA molecules such as mRNA molecules. The nucleic acid molecule may originate from genomic or expression libraries, generated from cells or organisms, e.g., eukaryotic or prokaryotic cells or organisms. This allows parallel sequencing of a plurality of different nucleic acid template molecules, e.g., at least 10, 100, 1.000 or 10.000 and up to 100.000, $10^6$ or $10^7$ or even more different nucleic acid molecules.

The nucleic acid molecules to be sequenced may be single-stranded nucleic acid molecules in a linear or circular form, e.g., in a covalently linked circular form. In order to obtain a circular nucleic acid template, a linear nucleic acid molecule may be subjected to a circularization procedure and optionally a strand-separation procedure during sample preparation. Circularization may be effected by ligation according to known protocols, e.g., using DNA or RNA ligases. In some embodiments, an adaptor and/or identifier molecule, i.e., a nucleic acid molecule of known sequence, may be coupled to the nucleic acid molecule.

The sequence determination may comprise nucleic acid elongation and/or nucleic acid degradation. The sequencing process includes one or more sequencing cycles.

The nucleic acid-synthesizing enzyme molecules are capable of elongating a primer annealed to a nucleic acid template molecule. Primer elongation may be carried out by progressively incorporating individual nucleotide building blocks at the 3'-terminus of a growing nucleic acid chain, wherein a nucleic acid molecule complementary to the sequence of the circular nucleic acid template is generated. The nucleic acid-synthesizing enzymes are selected from polymerases capable of a template specific nucleic acid polymerization, preferably from DNA polymerases and RNA polymerases, e.g., natural or modified polymerases, including thermostable DNA polymerases.

Specific examples of suitable DNA polymerases include Taq polymerases, exonuclease-deficient Taq polymerases, E. coli DNA polymerase I, Klenow fragment, reverse transcriptase, Φ29-related polymerases including wild-type Φ29 polymerase and derivatives of such polymerases, such as exonuclease-deficient forms, T7 DNA polymerase, T5 DNA polymerase, an RB69 polymerase and others.

Nucleic acid-degrading enzyme molecules are capable of progressively cleaving off individual nucleotide building blocks from a nucleic acid molecule. Preferably exonucleases, more preferably single-strand exonucleases which degrade in the 3'→5' direction or in the 5'→3' direction are used. Exonucleases which are particularly preferably used are 3'→5' exonucleases such as E. coli exonuclease I and E. coli exonuclease III, and 5'→3' exonucleases such as T7 exonuclease, E. coli exonuclease II and E. coli exonuclease VIII. Further, the exonuclease activities of various polymerases, e.g., the Klenow fragment, Taq polymerase or T4 polymerase may be used.

The nucleic acid-synthesizing enzyme molecules are contacted with a linear or circular nucleic acid template molecule, e.g., a single-stranded DNA or RNA molecule, and a primer molecule annealed to the nucleic acid template molecule or capable of annealing thereto. The primer molecule is preferably a single-stranded nucleic acid or nucleic acid analogue molecule having a free 3'-end which can be extended by an enzymatic reaction catalyzed by the immobilized nucleic acid-synthesizing enzyme molecules. The length of the primer molecule is selected to allow effective annealing to the template under reaction conditions. Usually, the length of the primer molecule is at least 8, at least 10, at least 12 or at least 15 nucleotides and e.g., up to 20, 25, 50 or 100 nucleotides, or even higher. In some embodiments, the primer is resistant against digestion by nucleic acid-degrading enzyme molecules, e.g., by incorporating nucleotide analogue building blocks and/or linkages between nucleotide building blocks, which are stable against degradation. In other embodiments, the primer is sensitive against digestion by nucleic acid-degrading enzyme molecules.

The sequence of the primer is selected in that it effectively anneals under reaction conditions to the template molecule. For instance, the primer may be a universal degenerated primer capable of statistically annealing to unknown nucleic acid sequences. In other embodiments, the primer may be capable of annealing to a known sequence portion of the nucleic acid template molecule. In this embodiment, a known adaptor and/or identifier sequence may be incorporated into the nucleic acid template molecule. The primer may be unlabelled or comprise fluorescent labelling groups.

Further, the presence of nucleotide building blocks carrying at least one fluorescent labelling group is required. Preferably, each different nucleotide building block (A, G, C, T/U) contains a different fluorescent labelling group.

The fluorescent labelling groups may be selected from known fluorescent labelling groups used for labelling biopolymers, particularly nucleic acids, such as, for example, fluorescein dyes, rhodamines, oxazines, for example Evoblue or Gnothis Blue, phycoerythrin, Cy3, Cy5, IR dyes or derivatives thereof, etc.

The nucleotide building blocks may carry (i) a fluorescence labelling group which remains with the building block when the building block is incorporated into a nucleic acid molecule during a primer elongation catalyzed by a nucleic acid-synthesizing enzyme molecule, and/or (ii) a fluorescence labelling group which is cleaved off from the building block when the building block is incorporated into a nucleic acid molecule during a primer elongation catalyzed by a nucleic acid-synthesizing enzyme molecule. Fluorescence labelling groups remaining with the building block are preferably attached to the α-phosphate group, to the sugar and/or to the nucleobase group.

In particular embodiments, fluorescence labelling groups remaining with the building block are attached to the nucleobase, e.g., via a linker which may have a chain-length of up to 15, preferably of 10-12 carbon atoms, optionally including heteroatoms, e.g., N, O or S atoms. Fluorescence labelling groups which are cleaved off when the building block is incorporated into a nucleic acid molecule may be attached to a terminal phosphate group, e.g., of a polyphosphate building block including, but not limited to a hexa-, penta-, tetra- or triphosphate building block such as the γ-phosphate group of a triphosphate building block. In certain embodiments, building blocks are selected which contain both (i) a fluorescence labelling group remaining after incorporation and (ii) a fluorescence labelling group cleaved off during incorporation. In this case, fluorescence groups capable of interacting with each other, e.g., by quenching and/or energy transfer, may be selected.

The nucleic acid molecules to be sequenced will contain fluorescent labelling groups in case the nucleic acid molecule is subjected to direct sequencing using a nucleic acid-degrading enzyme molecule. On the other hand, the nucleic acid molecule to be sequenced my not contain fluorescent labelling groups, if the nucleic acid molecule is used as a template in a primer elongation.

The sequencing procedure may involve a step of generating nucleic acid molecules having incorporated nucleotide building blocks in a primer elongation catalyzed by the nucleic acid-synthesizing enzyme molecules and/or a second step of cleaving off individual nucleotide building blocks from the generated nucleic acid molecules catalyzed by nucleic acid-degrading enzyme molecules. Dependent on the type of fluorescence labels, nucleic acid sequence determination may be carried out during primer elongation and/or during degradation.

Sequence determination during the primer elongation involves the use of nucleotide building blocks carrying a fluorescence-labelling group which is cleaved off from the building block when it is incorporated into a nucleic acid molecule. In this case, a time-dependent fluorescence change caused by cleaving off the fluorescence-labelling group from the nucleotide building block may be determined. Sequence determination during nucleic acid degradation involves the use of a nucleotide building block, which carries a fluorescence-labelling group which remains with the building block when it is incorporated into a nucleic acid molecule. Progressive cleavage of individual nucleotide building blocks from the nucleic acid molecules causes a time-dependent change of fluorescence when the labelled nucleotide building block is liberated from the nucleic acid molecule. In certain embodiments, it is also possible to carry out a sequence determination during elongation and degradation, i.e. when using nucleotide building blocks, which both carry a fluorescence-labelling group remaining with the building block and a fluorescence-labelling group which is cleaved off from the building block when the building block is incorporated into a nucleic acid molecule. In this embodiment, both fluorescent groups may be the same or different.

In some embodiments, the method involves one or more cycles of nucleic acid-synthesis and nucleic acid-degradation in order to determine the base sequence of a nucleic acid molecule template. The nucleic acid synthesis involves an elongation of the primer annealed to the nucleic acid template molecule catalyzed by the nucleic acid-synthesizing enzyme molecule, wherein a nucleic acid molecule complementary to the sequence of the nucleic acid template is generated. In the next step, the generated nucleic acid molecule is degraded by a nucleic acid-degrading enzyme molecule.

When a nucleotide building block is incorporated into an elongated nucleic acid molecule, a time dependent change in the fluorescence may occur, which can be detected as indicated above. Preferably, the incorporation of the nucleotide building blocks into the elongated nucleic acid molecule is associated with a detectable increase in the fluorescence, preferably with a transient increase in the fluorescence. For example, nucleotide building blocks may be used which carry a fluorescent labelling group on the portion of the molecule which is cleaved off when the building block is incorporated into the primer, e.g., on the γ-phosphate group.

When a nucleotide building block is cleaved off from the synthesized nucleic acid molecule, a time-dependent change of fluorescence may be determined due to the interaction of fluorescent labelling groups incorporated in nucleic acid strands with neighbouring groups, for example with chemical groups of the nucleic acids, in particular nucleobases such as, for example, G, or/and neighbouring fluorescent labelling groups, and these interactions leading to a change in fluorescence, in particular in fluorescence intensity, compared to the fluorescent labelling groups in "isolated" form, owing to quenching processes or/and energy transfer processes. The removal by cleavage of individual nucleotide building blocks alters the overall fluorescence, for example the fluorescence intensity of an immobilized nucleic acid strand, and this change is a function of the removal by cleavage of individual nucleotide building blocks, i.e., a function of time.

In certain embodiments, association of a labelled nucleotide with the biomolecule complex is detected by measuring polarisation of the emitted photons. The polarisation of excited states' photons is changed by the rotational movement of the light emitting nucleotide labels and can be used for identifying free moving contra bound labelled nucleotides in the polymerisation process.

This time-dependent change in fluorescence during elongation and/or degradation may be recorded in parallel for a multiplicity of nucleic acid molecules and correlated with the base sequence of the individual nucleic acid strands. Preference is given to using those fluorescent labelling groups which, when incorporated in the nucleic acid strand, are, at least partially, quenched so that the fluorescence intensity is increased after the nucleotide building block containing the labelling group or a neighbouring building block causing quenching has been removed by cleavage.

During incorporation and/or removal of individual nucleotide building blocks, it is possible to measure a change in fluorescence intensity of the nucleic acid strand or/and the incorporated or cleaved-off nucleotide building block, owing to quenching processes or energy transfer processes. This change in fluorescence intensity with time depends on the base sequence of the nucleic acid strand studied and can therefore be correlated with the sequence.

The complete sequence of the nucleic acid molecule may be determined by using a mixture of nucleotide building blocks, labelled on all four different bases, for example on A, G, C and T, or on combinations of two or three different bases. It is possible, where appropriate, to attach to the nucleic acid strand to be studied also a "sequence identifier", i.e., a labelled nucleic acid of known sequence, for example by enzymatic reaction using ligase or/and terminal transferase, so that at the start of sequencing initially a known fluorescence pattern and only thereafter the fluorescence pattern corresponding to the unknown sequence to be studied is obtained.

The detection comprises irradiating light into the support, preferably by means of a laser, or by another suitable light source, in order to cause excitation of the fluorescent labelling groups. It is possible, in this connection, to use one or more laser beams, for example an expanded laser beam, having a cross section of approx. 1-20 mm, and/or multiple laser beams. The detection preferably comprises a multipoint fluorescence excitation by lasers, for example a dot matrix of laser dots generated via diffraction optics (cf. WO 2002/097406) or a quantum well laser.

Fluorescence emission of a plurality of nucleic acid strands may be detected in parallel using a detector matrix which comprises, for example, an electronic detector matrix, for example a CCD camera, a CMOS detector matrix, e.g., a CMOS camera, or an avalanche photodiode matrix. The detection may be carried out in such a way that fluorescence excitation and detection are carried out in parallel on a part or all nucleic acid strands studied. Preference is given to carrying out the detection on fluorescence light which is emitted essentially orthogonally from the support surface through the reaction space or through the support body.

The detection may be carried out, for example, by means of a single molecule detection, for example by fluorescence correlation spectroscopy, which involves exposing a very small, preferably confocal, volume element, for example from $10^{-21}$ to $10^{-10}$ l, to the excitation light of a laser, or another suitable light source, which light excites the receptors present in this measuring volume so that the latter emit fluorescence light, the fluorescence light emitted from said measuring volume being measured by means of a photodetector and the change in the measured emission with time being correlated with the concentration of the analyte, so that it is possible to identify, at an appropriately high dilution, individual molecules in said measuring volume. Details of the procedure and of the apparatus used for detection can be found in the disclosure of EP 0 679 251, the content of which is herein incorporated by reference. The confocal determination of single molecules is furthermore described in Rigler and Mets (Soc. Photo-Opt. Instrum. Eng. 1921 (1993), 239 ff.) and Mets and Rigler (J. Fluoresc. 4 (1994) 259-264), the contents of which are herein incorporated by reference.

Alternatively, or additionally, detection may also be carried out by way of time-resolved decay measurement, called "time gating", as described, for example, by Rigler et al., "Picosecond Single Photon Fluorescence Spectroscopy of Nucleic Acids", in: "Ultrafast Phenomena", D.H. Auston, Ed., Springer 1984, the content of which is herein incorporated by reference. Here, the fluorescent molecules are excited in a measuring volume followed by, e.g., at a time interval of $\geq 100$ ps, opening a detection interval on the photodetector. In this way it is possible to keep background signals generated by Raman effects sufficiently low so as to enable single molecules to be detected in an essentially interference-free manner.

Further, the present disclosure is explained in detail by reference to the following specific embodiments.

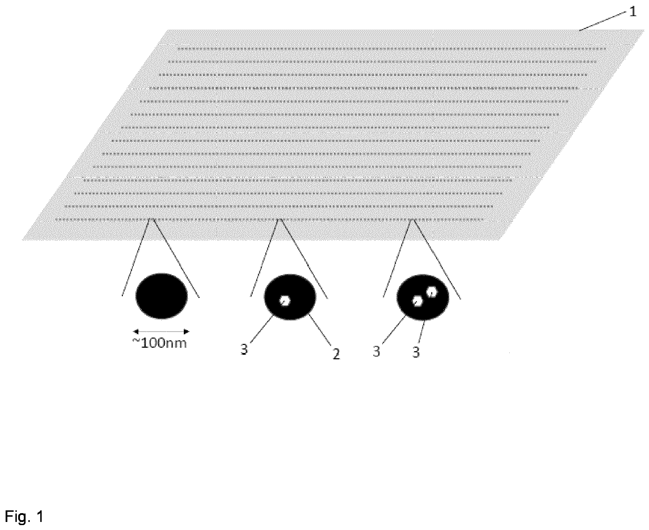

Fig. 1 shows an embodiment of the prior art. Biomolecules (3) are bound to sample spots (2) of about 100 nm diameter on a support (1). The molecules are bound stochastically, resulting in a Poisson distribution of the surface density of the bound molecules. According to this Poisson distribution, there is spectrum of sample spots without biomolecule bound thereto, sample spots with a single biomolecule bound thereto, and sample spots with two (or more) biomolecules bound thereto. (The parameters of the Poisson distribution depend on several parameters whereas the concentration of the biomolecules added in the step before binding is one such parameter).

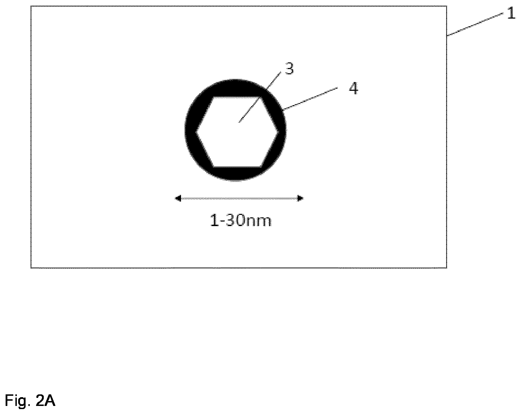

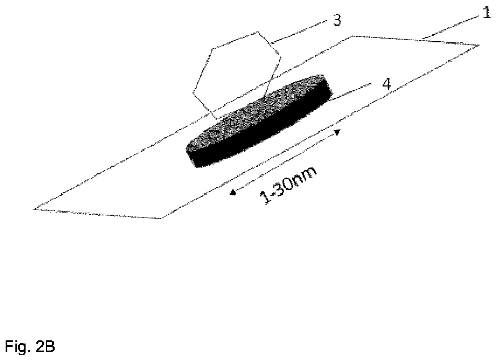

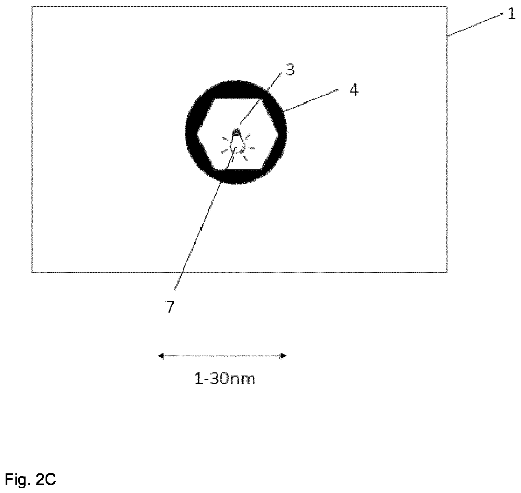

Fig. 2A, 2B and 2C show embodiments of the present disclosure.

A (top view) and B (side view): A sample spot (4) on a support (1) with a diameter of a single of 1-30 nm, particularly 5-10 nm and more particularly 6-8 nm, has a single biomolecule (3) attached thereto. Due to steric considerations with regard to size and/or surface chemistry of an individual spot, only one biomolecule (3) can be bound thereto. Performing the immobilization procedure of biomolecules under saturating conditions results in a high and (nearly) quantitative yield of spots to which one and only one biomolecule is bound.

C: A biomolecule (4) with a fluorescent group, e.g. an external labeling group attached to the biomolecule, is depicted. The fluorescent group may have an arbitrary emission wavelength distribution and a corresponding excitation wavelength distribution. In certain embodiments, the fluorescent group is visible during a limited time period, and in certain embodiment it is visible during a long time period. In another embodiment, the biomolecule does not need any external labeling group attached in order to detect the molecule by spectroscopic means, such as for example in the case of Raman spectroscopy.

**Claims**

1. A method for preparing a support for analyzing a single molecule event comprising the steps:

   (a) providing a support comprising a substrate and a plurality of sample spots on the surface of the support, wherein said sample spots are spatially separated from another and wherein said sample spots are adapted for the immobilization of one single biomolecule on one sample spot, and

(b) incubating said sample spots on said support with biomolecules under conditions that allow a saturating immobilization of said biomolecules to said sample spots.

2. The method of claim 1, wherein a single biomolecule is immobilized on a single sample spot in at least about 50%, at least about 60%, at least about 70% at least about 80%, at least about 90% or at least about 99% and up to 99.99% of said sample spots on said support.

3. The method of any one of the preceding claims, wherein the support comprises at least 10, at least 100, at least 1,000, at least $10^4$, at least $10^5$, at least $10^6$, at least $10^7$ or at least $10^8$ sample spots.

4. The method of any one of the preceding claims, wherein the sample spots have a size of about 5 nm to about 10 nm, particularly a size of about 6 nm to about 8 nm and more particularly about 7 nm for the immobilization of one single biomolecule.

5. The method of any one of the preceding claims, wherein the biomolecule is a nucleic acid-polymerizing enzyme, particularly a DNA or RNA polymerase, and/or a nucleic acid molecule, particularly a DNA or RNA molecule.

6. The method of any one of the preceding claims, wherein said sample spots are adapted in their surface chemistry for the immobilization of one single biomolecule on one said sample spot.

7. The method of claim 6, wherein the surface of the sample spots comprises at least one functional anchor molecule, which comprises a moiety suitable for the direct or indirect attachment of a biomolecule.

8. The method of claim 7, wherein said functional anchor molecule is attached to the surface of said sample spots with sulfur-containing groups, e.g., thiol groups.

9. The method of claim 7 or 8, wherein the sample spots comprise a metal surface, particularly an Au or Au/Pd surface.

10. The method of any one of the preceding claims, wherein the conditions that allow a saturating immobilization of said biomolecules to said sample spots comprise adapting at least one of the following parameters:

(i) the concentration of biomolecules;
(ii) the incubation time;
(iii) the incubation temperature;
(iv) any combination of (i), (ii), and/or (iii).

11. The method of any one of the preceding claims, wherein the conditions that allow a saturating immobilization of said biomolecules to said sample spots comprise carrying out the incubation step under conditions wherein the amount of biomolecules is higher than the amount of available sample spots.

12. A support comprising a substrate and a plurality of sample spots on the surface of the support, wherein the sample spots are spatially separated from another, and wherein a biomolecule is immobilized on a sample spot, wherein a single biomolecule is immobilized on a single sample spot in at least about 50% of said sample spots.

13. Use of the support of claim 12 for analyzing a single molecule event, particularly for the sequence analysis of a single nucleic acid molecule.

14. A method for analyzing a single molecule event comprising:

(i) providing a support of claim 12, wherein a single biomolecule is immobilized on a single sample spot in at least about 50% of said sample spots, and
(ii) analyzing an event, associated with said single biomolecule, by detecting electromagnetic radiation from an individual sample spot.

15. A device for analyzing a single molecule event comprising:

(i) a support of claim 12, wherein a single biomolecule is immobilized on a single sample spot in at least about 50% of said sample spots,
(ii) means for illuminating at least one sample spot on the support with radiation, and
(iii) means for analyzing an event associated with said single biomolecule, by detecting electromagnetic radiation from an individual sample spot.

Fig. 1

Fig. 2A

Fig. 2B

Fig. 2C

Europäisches
Patentamt

European
Patent Office

Office européen
des brevets

## EUROPEAN SEARCH REPORT

Application Number

EP 22 21 1669

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/041095 A1 (ROTHBERG JONATHAN M [US] ET AL) 11 February 2016 (2016-02-11) * abstract * * figures 10-1 to 10-23 * * paragraphs [0197], [0224] * * paragraph [0207] - paragraph [0208] * * paragraph [0547] - paragraph [0550] * * claims 34-62 * ----- | 1-15 | INV. C12Q1/6806 C12Q1/6869 G01N21/64 |
| X | EP 3 112 841 A1 (GNOTHIS HOLDING AG) 4 January 2017 (2017-01-04) * paragraph [0012] - paragraph [0017] * * paragraph [0019] - paragraph [0020] * * paragraph [0078] - paragraph [0079] * * claims 1-4,13-14 * * figures 1-2 * ----- | 1-15 | |
| X,D | WO 2015/104245 A1 (GNOTHIS HOLDING AG [CH]) 16 July 2015 (2015-07-16) | 1-6,9, 10,12-15 | |
| A | * abstract * * claims 2,6,10 * * figures 5-6 * ----- | 7,8,11 | TECHNICAL FIELDS SEARCHED (IPC) |
| A,D | WO 2013/131888 A1 (RIGLER RUDOLF [SE]) 12 September 2013 (2013-09-12) * claims 1-27 * ----- | 1-15 | G01N C12Q |

The present search report has been drawn up for all claims

| Place of search | Date of completion of the search | Examiner |
|---|---|---|
| Munich | 20 May 2023 | Barz, Wolfgang |

EPO FORM 1503 03.82 (P04C01)

## ANNEX TO THE EUROPEAN SEARCH REPORT
## ON EUROPEAN PATENT APPLICATION NO.

EP 22 21 1669

This annex lists the patent family members relating to the patent documents cited in the above-mentioned European search report.
The members are as contained in the European Patent Office EDP file on
The European Patent Office is in no way liable for these particulars which are merely given for the purpose of information.

20-05-2023

| Patent document cited in search report | | | Publication date | Patent family member(s) | | | Publication date |
|---|---|---|---|---|---|---|---|
| US 2016041095 | A1 | | 11-02-2016 | AU | 2015300778 | A1 | 16-03-2017 |
| | | | | BR | 112017002489 | A2 | 05-12-2017 |
| | | | | CA | 2957543 | A1 | 11-02-2016 |
| | | | | CN | 106796175 | A | 31-05-2017 |
| | | | | EP | 3194933 | A1 | 26-07-2017 |
| | | | | JP | 6812341 | B2 | 13-01-2021 |
| | | | | JP | 2017531168 | A | 19-10-2017 |
| | | | | KR | 20170041848 | A | 17-04-2017 |
| | | | | KR | 20220165282 | A | 14-12-2022 |
| | | | | US | 2016041095 | A1 | 11-02-2016 |
| | | | | US | 2018231465 | A1 | 16-08-2018 |
| | | | | US | 2019383739 | A1 | 19-12-2019 |
| | | | | US | 2022120685 | A1 | 21-04-2022 |
| | | | | WO | 2016023010 | A1 | 11-02-2016 |
| EP 3112841 | A1 | | 04-01-2017 | CN | 108124455 | A | 05-06-2018 |
| | | | | EP | 3112841 | A1 | 04-01-2017 |
| | | | | EP | 3317637 | A1 | 09-05-2018 |
| | | | | JP | 6871181 | B2 | 12-05-2021 |
| | | | | JP | 2018526979 | A | 20-09-2018 |
| | | | | US | 2018187258 | A1 | 05-07-2018 |
| | | | | WO | 2017001407 | A1 | 05-01-2017 |
| WO 2015104245 | A1 | | 16-07-2015 | CN | 106460034 | A | 22-02-2017 |
| | | | | EP | 3092314 | A1 | 16-11-2016 |
| | | | | JP | 6731854 | B2 | 29-07-2020 |
| | | | | JP | 2017504039 | A | 02-02-2017 |
| | | | | US | 2016326581 | A1 | 10-11-2016 |
| | | | | US | 2022002800 | A1 | 06-01-2022 |
| | | | | WO | 2015104245 | A1 | 16-07-2015 |
| WO 2013131888 | A1 | | 12-09-2013 | EP | 2823056 | A1 | 14-01-2015 |
| | | | | US | 2015080227 | A1 | 19-03-2015 |
| | | | | US | 2018223355 | A1 | 09-08-2018 |
| | | | | WO | 2013131888 | A1 | 12-09-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2002097406 A
- WO 2003052137 A
- WO 2006013110 A
- WO 2013131888 A
- WO 2015104245 A
- WO 2017001407 A
- WO 2018104301 A
- US 63382624 B
- US 7745116 B2
- EP 0679251 A

**Non-patent literature cited in the description**

- **RIGLER ; METS.** *Soc. Photo-Opt. Instrum. Eng.,* 1993, vol. 1921, 239
- **METS ; RIGLER.** *J. Fluoresc.,* 1994, vol. 4, 259-264
- Picosecond Single Photon Fluorescence Spectroscopy of Nucleic Acids. **RIGLER et al.** Ultrafast Phenomena. Springer, 1984